# EUROPEAN PATENT SPECIFICATION

(11) **EP 2 506 867 B1**
(45) Date of publication and mention of the grant of the patent: **08.10.2014**
(21) Application number: 10790375.9
(22) Date of filing: 02.12.2010
(51) Int. Cl.: A61K 38/18, A61K 38/30, A61K 38/48, A61K 38/22, A61K 38/19, A61K 31/529, A61K 45/06, A61P 9/10

(54) **PHARMACEUTICAL COMPOSITIONS FOR THE STIMULATION OF STEM CELLS.**
PHARMAZEUTISCHE ZUSAMMENSETZUNGEN ZUR STIMULATION VON STAMMZELLEN
COMPOSITIONS PHARMACEUTIQUES POUR LA STIMULATION DES CELLULES SOUCHES

(30) Priority: 02.12.2009 WO PCT/EP2009/066251
(43) Date of publication of application: 10.10.2012
(73) Proprietor: Cardio3 BioSciences S.A., 1435 Mont-Saint-Guibert (BE)
(72) Inventor: GORDON-BERESFORD, Roland, B-1310 La Hulpe (BE); GAUSSIN, Vinciane, B-1970 Wezembeek-oppem (BE); LATERE DWAN'ISA, Jean-Pierre, B-5000 Namur (BE); HOMSY, Christian, B-1150 Bruxelles (BE)
(74) Representative: Crease, Devanand John
(86) International application number: PCT/EP2010/068700
(87) International publication number: WO 2011/067317

(56) References cited:
- WO-A2-2006/015127
- WO-A2-2006/081190
- WO-A2-2009/151907
- US-A1- 2008 019 944
- US-A1- 2009 155 344
- BARTUNEK JOZEF ET AL: "Pretreatment of adult bone marrow mesenchymal stem cells with cardiomyogenic growth factors and repair of the chronically infarcted myocardium", AMERICAN JOURNAL OF PHYSIOLOGY - HEART AND CIRCULATORY PHYSIOLOGY, vol. 292, no. 2, February 2007 (2007-02), pages H1095-H1104, XP002592697, ISSN: 0363-6135
- TORELLA DENISE ET AL: "Cardiac stem cells regenerate the infarcted heart, restoring function and long-term survival in mice", CIRCULATION, vol. 110, no. 17, Suppl. S, October 2004 (2004-10), page 170, XP009136301, & 77TH SCIENTIFIC MEETING OF THE AMERICAN-HEART-ASSOCIATION; NEW ORLEANS, LA, USA; NOVEMBER 07 -10, 2004 ISSN: 0009-7322
- BEHFAR ATTA ET AL: "Cardiopoietic programming of embryonic stem cells for tumor-free heart repair", THE JOURNAL OF EXPERIMENTAL MEDICINE, ROCKEFELLER UNIVERSITY PRESS, US, vol. 204, no. 2, 1 February 2007 (2007-02-01), pages 405-420, XP002462528, ISSN: 0022-1007, DOI: DOI:10.1084/JEM.20061916
- TOKUNOU TOMOTAKE ET AL: "Engineering a new insulin-like growth factor-1 protein for embryonic stem cell therapy", CIRCULATION, LIPPINCOTT WILLIAMS & WILKINS, US, vol. 114, no. 18, suppl. S, 1 October 2006 (2006-10-01), page 239, XP009124938, ISSN: 0009-7322
- BEHFAR ATTA ET AL: "Guided Cardiopoiesis Enhances Therapeutic Benefit of Bone Marrow Human Mesenchymal Stem Cells in Chronic Myocardial Infarction", JOURNAL OF THE AMERICAN COLLEGE OF CARDIOLOGY, vol. 56, no. 9, August 2010 (2010-08), pages 721-734, XP002623549, ISSN: 0735-1097

## Description

### TECHNICAL FIELD

The present invention relates generally to pharmaceutical compositions suitable for targeting tissues and/or organs. In particular, it relates to the treatment of heart diseases through administration of stem cell-stimulating agents to the heart of an individual in need of heart tissue regeneration. In particular, it discloses the use of such stem cell-stimulating agents to improve the regeneration of cardiac tissue from cardiac stem cells *in vivo.*

### DESCRIPTION OF RELATED ART

Myocardial infarction (MI) results in loss of cardiomyocytes, scar formation, ventricular remodeling, and eventually heart failure. Pharmacologic, catheter-based, and surgical interventions have led to improved survival of patients with coronary artery disease (CAD), although they fail to regenerate dead myocardium. Consequently, reduced mortality is accompanied by increased morbidity because of ischemic heart failure. In recent years, stem cell-based therapy has emerged as a potential new strategy for cardiac repair (Dimmeler S. et al., J Clin Invest 2005, 11, 572-583). The optimal source of cells for repairing damaged myocardium is a topic of intense research. Important features of stem cells for cardiac regeneration include self renewal, clonogenicity, and the ability to differentiate into cardiomyocytes, endothelial cells and vascular smooth muscle cells.

Over the past 10 years, researchers have applied various bone marrow (BM)-derived stem/progenitor cells for cardiac reparative therapy in animal studies, such as lineage negative (*lin* neg) c-kit positive (c-kit pos) BM stem cells, (Orlic et al., Nature 2001 ; 410: 701-705; Kajstura et al., Circ. Res. 2005; 96: 127-137; Rota et al., Proc Natl Acad Sci USA 2007 ; 104: 17783-17788) BM-derived mesenchymal stem cells (MSCs) (Min et al., Ann Thor Surg 2002; 74: 1568-1575; Amado et al., Proc Natl Acad Sci USA 2005 ; 102: 11474-11479) and endothelial progenitor cells (EPCs) (Cho et al., J Exp Med 2007; 204: 3257-3269; Schuh et al., Basic Res Cardiol 2008; 103:60-77). Despite these studies showing the differentiation of BM-derived stem/progenitor cells into cells with hallmark features of cardiomyocytes and vascular cells, other studies suggested that the transplanted BM stem cells do not readily acquire a cardiac phenotype in the injured heart (Balsam et al., Nature 2004; 428: 668-673; Murry et al., Nature 2004; 428: 664-668; Nygren et al., Nat Med 2004; 10: 494-501).

Thus, enhancing differentiation of BM-derived stem/progenitor cells after their transplantation remains a great challenge for researchers to effectively use these cells in cardiac regenerative therapy. Other stem cell sources may be used for cardiac regenerative therapy apart from BM-derived stem/ progenitor cells. In particular, resident cardiac stem cells (CSCs) were discovered in the heart itself (Beltrami et al., Cell 2003; 114: 763-776; Uranek et al., Proc Natl Sci USA 2006; 103: 9226-9231). Because CSCs already reside within the heart and are programmed to generate cardiac tissue, they represent a logical source to exploit in cardiac regenerative therapy, when massive loss of cardiac tissue occurred. Given that CSCs have unique characteristics, the identification of resident CSCs created great excitement and sparked intense hope for myocardial regeneration with cells that are from the heart itself and are thereby inherently programmed to reconstitute cardiac tissue.

Myocardial repair requires the formation of new myocytes and coronary vessels, and it cannot be accomplished by a cell already fully committed to the myocyte lineage. In the presence of an infarct, the generation of myocytes alone cannot restore contractile performance in the akinetic region; myocytes will not grow or survive in the absence of vessel formation. Arterioles are critical for blood supply, and oxygen delivery is controlled by the capillary network. Similarly, neovasculogenesis alone would not restore the dead myocardium or reinstitute contractile activity in the infarcted portion of the ventricular wall. Observation that CSCs injected locally in the infarcted myocardium of animals repaired the necrotic tissue and improve ventricular function (Beltrami et al., Cell 2003; 114: 763-776; Bearzi et al., Proc Natl Sci USA 2007 ; 104 : 14068-14073) has formed the basis of a new paradigm in which CSCs are implicated in the normal renewal of myocytes, endothelial cells, smooth muscle cells, and fibroblasts. In an attempt to develop strategies relevant to the future treatment of patients, new hypotheses have to be raised to move the field in a direction that defines CSCs therapy clinically on an individual basis.

Various attempts have thus been made to deal with the discovered CSCs for clinical applications. The first approach is isolation, culture, cloning and expansion of CSCs. Such cells would be injected back into the infracted heart in an attempt to regenerate functional myocardium. However, the scarcity of the CSC cell population, combined to stringent cell culture conditions and poor yield, are limiting factors using this approach. The other alternative described in the art is the recruitment and differentiation of endogenous CSCs using exogenous agents. However, no clear evidence on the efficacy of this approach has been described casting uncertainty on the capacity to effectively recruit and differentiate CSCs in vivo.

### SUMMARY OF THE INVENTION

The present invention provides a totally novel approach to stimulate in vivo resident CSCs and, in one aspect, to commit them into the cardiac lineage, particularly to obtain from them a significant number of satisfactorily functional cells with hallmark features of cardiomyocytes.

The invention relates to a human or veterinary pharmaceutical composition (B) for the stimulation of stem cells as claimed.

Said stem cells-stimulating-agents are TGFβ-1, BMP-4, FGF-2, IGF-1, Activin-A Cardiotrophin 1 and Cardiogenol C.

The invention also relates to a pharmaceutical cocktail comprising a pharmaceutical composition (B) according to the present invention and a composition (A) comprising at least one pharmaceutically active substance. The composition or cocktail of the present invention allows to provide stimulating agent-guided stem cells which means that resident cardiac stem cells, after having been put into contact with the composition or cocktail, are stimulated to enter into differentiation. Hence, the stimulated stem cells may be committed into a cardiac lineage and may become a cardiomyocyte.

Within the frame of the present document, and unless indication of the contrary, the terms designated below between quotes have the following definitions.

As used herein, the term "stimulation or stimulating" refers to recruitment, proliferation, survival and/or differentiation of stem cells.

As used herein, the terms "cardiac tissue" and "myocardium" refer to myocytes, blood vessels and fibroblasts.

'Cardiac stem cells' (CSCs), 'cardiac progenitor cells' , 'resident cardiac stem cells' or 'resident cardiac progenitor cells' designate stem cells which are present in the myocardium. They are self-renewing, clonogenic, multipotent and may generate myocardium.

A 'stem cells stimulating agent' is an agent which improves the ability of stem cells, to be recruited to the site to be regenerated, to proliferate and to differentiate into cardiac tissue.

A 'stem cells stimulating agent composition' is a composition comprising at least two stem cell stimulating agents.

A 'stimulating agent-guided stem cell' is a stem cell which was in contact with a stem cell stimulating agent composition as defined above and further enters into differentiation i.e. is committed into the cardiac lineage.

The 'differentiation' is the process by which a less specialized cell becomes a more specialized cell.

Unless otherwise defined, all technical and scientific terms used herein have the same meaning as commonly understood by one of ordinary skill in the art to which this invention pertains. Although methods and materials similar or equivalent to those described herein can be used to practice the disclosure, suitable methods and materials are described bellow. In case of conflict, the present specification, including definitions, will control the meaning of terms of the present invention. In addition, the materials, methods, and examples are illustrative only and not intended to be limiting.

### Detailed description of the invention

The pharmaceutical composition (B) comprises stem cells-stimulating-agents which are TGFβ-1, BMP-4, FGF-2, IGF-1, Activin-A, Cardiotrophin 1 and Cardiogenol C.

Said pharmaceutical composition (B) may further comprise thrombin inhibitors, such as hirudin, bivalirudin, lepirudin, deirudin, argatroban, melagatran, ximelagatran, dabigatran, and heparin. Alpha-thrombin is a coagulant agent. Said pharmaceutical composition (B) is free of alpha-thrombin.

The pharmaceutical composition (B) of the present invention may further comprise at least one substance selected in the group consisting of growth factors, cytokines, hormones and combinations thereof. Said at least one substance may be selected in the group consisting of:
- Bone morphogenetic proteins (BMP) such as BMP-1, BMP-2, BMP-5, BMP-6;
- Epidermal growth factor (EGF);
- Erythropoietin (EPO);
- Fibroplast growth factors (FGF) such as FGF-1, FGF-4, FGF-5, FGF-12, FGF-13, FGF-15, FGF-20;
- Granulocyte-colony stimulating factor (G-CSF);
- Granulocyte-macrophage colony stimulating factor (GM-CSF);
- Growth differentiation factor-9 (GDF-9);
- Hepatocyte growth factor (HGF);
- Insuline-like growth factor (IGF) such as IGF-2;
- Myostatin (GDF-8);
- Neurotrophins such as NT-3, NT-4, NT-1 and Nerve growth factor (NGF) ;
- Platelet-derived growth factor (PDGF) such as PDGF-beta, PDGF-AA, PDGF-BB;
- Thrombopoietin (TPO);
- TGF- (Transforming growth factor alpha)
- Transforming growth factors β, (TGF- β) such as TGF-β1, TGF-β2, TGF-β3;
- VEGF (Vascular endothelial growth factor) such as VEGF-A, VEGF-C;
- TNF-α, Leukemia inhibitory factor (LIF), interleukin 6 (IL-6), retinoic acid, C SDF-1 (stromal cell-derived factor-1), BDNF (brain-derived neurotrophic factor), Periostin, Angiotensin II, Flt3 Ligand, Glial-Derived Neurotrophic Factor, Heparin, Insulin-Like Growth Factor Binding Protein-3, Insulin- Like Growth Factor Binding Protein-5, Interleukin-3, Interleukin-8, Midkine, Progesterone, Putrescine, Stem Cell Factor, TGF-alpha, Wntl, Wnt3a, Wnt5a, caspase-4, chemokine ligand 1, chemokine ligand 2, chemokine ligand 5, chemokine ligand 7, chemokine ligand 11, chemokine ligand 20, haptoglobin, lectin, cholesterol 25-hydroxylase, syntaxin-8, syntaxin-11, ceruloplasmin, complement component 1, complement component 3, integrin alpha 6, lysosomal acid lipase 1, ϑ-2 microglobulin, ubiquitin, macrophage migration inhibitory factor, cofilin, cyclophillin A, FKBP12, NDPK, profilin 1, cystatin C, calcyclin, stanniocalcin-1, PGE-2, mpCCL2, IDO, iNOS, HLA-G5, M-CSF, angiopoietin, PIGF, MCP-1, extracellular matrix molecules, CCL2 (MCP-1), CCL3 (MIP-1α), CCL4 (MIP-1β), CCL5 (RANTES), CCL7 (MCP-3), CCL20 (MIP-3α), CCL26 (eotaxin-3), CX3CL1 (fractalkine), CXCL5 (ENA-78), CXCL11 (i-TAC), CXCL1 (GROα), CXCL2 (GROβ), CXCL8 (IL-8), CCL10 (IP-10) and combinations thereof.

The stem cells to be stimulated may be resident cardiac stem cells (CSCs) or circulating stem cells or injected stem cells.

Said pharmaceutical composition may comprise primary particles. Said primary particles may be selected from the group consisting of alginates, chitosan, dextran, cellulose, liposome, or microspheres or nanospheres of polyesters such as PLGA, polycaprolactone or copolyesters. Preferably, said primary particles may encapsulate said at least two stem-cells-stimulating agents of said pharmaceutical composition (B). Hence, said primary particles may encapsulate the stem cells-stimulating agents comprised in the pharmaceutical composition (B). The term "primary" means that the pharmaceutical composition may be encapsulated in a first type of particles as defined above.

Preferably, said pharmaceutical composition (B) may be combined with a composition (A) comprising at least one pharmaceutically active substance to form a pharmaceutical cocktail. In one embodiment, said at least one pharmaceutically active substance may be selected in the group consisting of insulin-like growth factor 1 (IGF-1), hepatocyte growth factor (HGF) and/or variants thereof such as NK1, 1K1, 1K2, HP11, or HP21, and combinations thereof. In another embodiment, said composition (A) may further comprise SCF-1. Said composition (A) may further comprise secondary particles selected from the group consisting of alginates, chitosan, dextran, cellulose, liposomes, or microspheres or nanospheres of polyesters such as PLGA, polycaprolactone or copolyesters. Said secondary particles may encapsulate said at least one pharmaceutically active substance. The term "secondary" means that the composition (A) may be encapsulated in a second type of particles as defined above. In addition, said secondary particles may be configured to allow a delivery of the substances encapsulated therein before the delivery of the substance encapsulated in primary particles.

Said pharmaceutical cocktail may comprise a sample of said composition (B) and a sample of said composition (A). Alternatively, both compositions (A) and (B) may be mixed together in a single sample. When mixed together, compositions (A) and (B) may, however, be administrated to or delivered in the area, or surrounding the area, to be treated separately.

Said pharmaceutical composition of the present invention or said pharmaceutical cocktail may be used as medicine. Alternatively, said pharmaceutical composition of the present invention or said pharmaceutical cocktail may be used for the regeneration of cardiac tissue. Alternatively, said pharmaceutical composition of the present invention or said pharmaceutical cocktail may be used for the treatment of heart disease, including heart failure, heart ischemia or myocardial infarction.

In another aspect of the present disclosure, a process for acting *in vivo* or *ex vivo* on CSCs of human or animals is provided. Said process comprises the step of administrating said pharmaceutical composition (B) or said pharmaceutical cocktail of the present invention to said humans or animals.

The administration of the pharmaceutical composition (B) may follow a preliminary administration of a composition (A) comprising at least one pharmaceutically active substance.

The administration may be performed by sequential injection of composition A, B or of the cocktail.

Moreover, the duration between two successive administrations of said pharmaceutical composition or pharmaceutical cocktail of the present invention may be from one hour to 180 days. Each administration may be repeated. Alternatively, each or some administrations of said composition (A) may be optional.

Hence, said pharmaceutical composition (B) or pharmaceutical cocktail may be administrated parenterally. Moreover, said pharmaceutical composition (B) or pharmaceutical cocktail may be administrated into the circulatory system of a human or animal. Said pharmaceutical composition (B) or pharmaceutical cocktail may be administrated into veins and/or arteries.

The pharmaceutical composition (B) or pharmaceutical cocktail of the present invention may be administrated to a cardiac tissue. In the preferred embodiment, the administration may be intracoronary for said pharmaceutical composition (B) and intravenous for said preliminary administration of the composition (A).

TGFβ as used herein refers to TGFβ-1, TGFβ-2 or TGFβ-3 and can be any polypeptide having TGFβ activity, such as human TGFβ. For example, TGFβ can be recombinant TGFβ. In one embodiment, TGFβ can be TGFβ-1. Any appropriate concentration of TGFβ can be used. For example, between 0.1 and 100 ng of TGF-β per ml (e.g., about 33 ng of TGFβ1 per ml) can be used.

BMP can be any polypeptide having BMP activity, such as human BMP. For example, BMP can be recombinant BMP. In one embodiment, BMP can be BMP4. Any concentration of BMP can be used. For example, between 0.1 and 200 ng of BMP per ml (e.g., about 65 ng of BMP4 per ml) can be used.

FGF-2 can be any polypeptide having FGF-2 activity, such as human FGF-2. For example, FGF-2 can be recombinant FGF-2. Any concentration of FGF-2 can be used. For example, between 0.1 and 200 ng of FGF-2 per ml (e.g., about 65 ng of FGF-2 per ml) can be used.

IGF-1 can be any polypeptide having IGF-1 activity, such as human IGF-1. For example, IGF-1 can be recombinant IGF-1. Any concentration of IGF-1 can be used. For example, between 1 and 1000 ng of IGF-1 per ml (e.g., about 650 ng of IGF-1 per ml) can be used.

Activin-A can be any polypeptide having Activin-A activity, such as human Activin-A. For example, Activin-A can be recombinant Activin-A. Any concentration of Activin-A can be used. For example, between 0.1 and 500 ng of Activin-A per ml (e.g., about 130 ng of Activin-A per ml) can be used.

α-Thrombin can be any polypeptide having α-thrombin activity, such as human α-thrombin. For example, α-thrombin can be recombinant α-thrombin or synthetic α-thrombin.

Cardiotrophin can be any polypeptide having Cardiotrophin activity, such as human Cardiotrophin-1. For example, Cardiotrophin can be recombinant Cardiotrophin. Any concentration of Cardiotrophin can be used. For example, between 0.05 and 100 ng of Cardiotrophin per ml (e.g., about 13 ng of Cardiotrophin-1 per ml) can be used.

IL-6 can be any polypeptide having IL-6 activity, such as human IL-6. For example, IL-6 can be recombinant IL-6. Any concentration of IL-6 can be used. For example, between 10 and 400 ng of IL-6 per ml can be used.

Any concentration of Cardiogenol C or a pharmaceutically acceptable salt thereof (e.g., Cardiogenol C hydrochloride) can be used. For example, between 1 and 1000 ng of Cardiogenol C per ml (e.g., about 350 ng per ml of Cardiogenol C) can be used.

Retinoic acid can be any molecule having retinoic acid activity, such as synthetic retinoic acid, natural retinoic acid, a vitamin A metabolite, a natural derivative of vitamin A, or a synthetic derivative of vitamin A. Any concentration of retinoic acid can be used. For example, between 1 x 10⁻⁷ and 4 x 10⁻⁶ µM of retinoic acid can be used.

In some cases, serum-containing or serum-free media supplemented with TGFβ-1 (e.g., 2.5 ng/ml), BMP4 (e.g., 5 ng/ml), FGF-2 (e.g., 5 ng/ml), IGF-1 (e.g., 50 ng/ml), Activin-A (e.g., 10 ng/ml), Cardiotrophin (e.g., 1 ng/ml), α-thrombin (e.g., 1 Unit/ml), and Cardiogenol C (e.g., 100 nM) can be used. In some cases, the media (e.g., serum-containing or serum-free media) can contain platelet lysate (e.g., a human platelet lysate).

In some cases, the composition used to stimulate CSCs may contain additional optional factors such as TNF-α, LIF, and VEGF-A.

TNF-α can be any polypeptide having TNF-α activity, such as human TNF-α. For example, TNF-α can be recombinant TNF-α. Any concentration of TNF-α can be used. For example, between 0.5 and 100 ng of TNF-α per ml can be used.

LIF can be any polypeptide having LIF activity, such as human LIF. For example, LIF can be recombinant LIF. Any concentration of LIF can be used. For example, between 0.25 and 200 ng of LIF per ml can be used.

VEGF-A can be any polypeptide having VEGF-A activity, such as human VEGF-A. For example, VEGF-A can be recombinant VEGF-A. Any concentration of VEGF-A can be used. For example, between 0.5 and 400 ng of VEGF-A per ml can be used.

A composition provided herein can be prepared using any appropriate method. For example, a composition provided herein can be prepared using commercially available stimulating agents. In some cases, a composition provided herein can be prepared to contain cells lysates (e.g., a platelet lysate) or conditioned media from cells such as cardiomyocyte cells or TNF-α-stimulated endodermal cells. For example, a composition provided herein can be prepared using a platelet lysate supplemented with commercially available factors. In some cases, a composition provided herein can be prepared using factors isolated from conditioned medium. In some cases, the factors can be dissolved in media such as cell culture media that does or does not contain serum.

### Examples

Tests are performed in acutely infarcted pigs. The following protocol is established. The infarct is performed at T0 by 90 minutes left anterior descending (LAD) occlusion followed by a 30 minutes reperfusion. At the end of the reperfusion (T1), a primary composition is parenterally administrated to the animals by intracoronary delivery distal to the occlusion site. A BrdU loaded osmotic pump is also subcutaneously implanted at T1. Fourteen days later (T2), a secondary composition is parenterally administrated to the animals. The administration of both compositions can be achieved with different methods of administration such as intravenous injection, intramuscular injection or intracoronary injection. Finally, at 42 days (T3), the euthanasia of the pigs is performed.

The concentration of constituents are mentioned in brackets. Two compositions, alone or in combination, are tested:
- Composition A consists of IGF-1 (8 µg in 15ml of Phosphate Buffer Solution (PBS)) and HGF (2 µg in 15 ml of PBS) and
- Composition B consists of TGFβ-1 (0.5 µg in 15ml of PBS), BMP4 (1 µg in 15ml of PBS), FGF-2 (1 µg in 15ml of PBS), IGF-1 (10 µg in 15ml of PBS), Activin-A (2 µg in 15ml of PBS), Cardiotrophin 1 (0.2 µg in 15ml of PBS) and Cardiogenol C (5.2 µg in 15ml of PBS).
Both compositions are in a pharmaceutically acceptable excipient. The pharmaceutically acceptable excipient may be phosphate buffered solution (PBS), Hartmann's solution, Ringer's lactate, physiological NaCl (0.9% NaCl) supplemented or not with albumin or with any suitable protein stabilizer composition.

Five treatment groups of 5 animals each are evaluated.
- Treatment group 1 is a control group and only receives saline solution at T1 and T2.
- Treatment group 2 receives a solution containing the composition A at T1 and a saline solution at T2, noted as mix A.
- Treatment group 3 receives a solution containing the composition A at T1 and a solution containing the composition B at T2, noted mix A+B.
- Treatment group 4 receives a solution containing the composition B at T1 and T2, noted mix B+B.
- Treatment group 5 receives a solution containing the composition B at T1 and a saline solution at T2, noted mix B.

The protocol is summarized in table 1.

**Table 1**

| | Treatment group 1 | Treatment group 2 | Treatment group 3 | Treatment group 4 | Treatment group 5 |
|---|---|---|---|---|---|
| T0: infarct | | | | | |
| T1: 30min | Saline | A | A | B | B |
| T2: 2 weeks | Saline | Saline | B | B | Saline |
| T3: 6 weeks | Euthanasia | Euthanasia | Euthanasia | Euthanasia | Euthanasia |

Blood analyses were performed at different intervals. Blood samples are collected from 2 pigs per treatment group in coronary sinus via jugular vein and venous blood via ear vein. After the primary administration, samples are collected at T1+5min; T1+1h and T1+6h. After the secondary administration, samples are collected at T2+5min; T2+1 h, T2+6h and T2+24h. ELISA immunoassays are performed with samples for the quantification of IGF-1 and cardiotrophin 1 concentration.

Magnetic resonance imaging (MRI) is also performed on all animals at T1+3 days; T2 and T3 to study the scar area, the global left ventricular function, the regional function (wall motion and thickening) and regional perfusion of the ventricular. MRI allows to detect and confirm the presence of new vessels, tissue or cells improving ventricular function.

Histopathology is also performed to determine the scar area, the identification and quantification of c-kit positive cardiac stem cells. Histopathology also provides data on distribution, size and density of new vessels and cardiomyocytes. Histopathology allows documenting the repair process at the tissue and cellular level.

Critical variables have been considered in the analysis of cardiac repair: (1) amount of reconstituted tissue or myocardium mass and coronary vasculature; (2) number and size of restored myocytes and vessels; (3) integration of newly formed myocytes and vessels with the surrounding myocardium; and/or (4) origin of the regenerated myocardial structures.

### Infarct result

Images from MRI imaging were used to evaluate infarct size, infarct weight and the infarct area. Results are listed in Table 2 below.

**Table 2**

| Infarct | Infarct area (%) | Infarct weight (g) | Infarct Volume (cm³) |
|---|---|---|---|
| Group 1 (Control) | 19.8 | 15.6 | 22.0 |
| Group 2 (Mix A) | 18.7 | 15.8 | 20.7 |
| Group 5 (Mix B) | 13.7 | 13.2 | 15.6 |
| Group 4 (Mix B+B) | 18.8 | 22.2 | 25.2 |
| Group 3 (Mix A+B) | 9.6 | 10.3 | 10.3 |

Experiments demonstrate that the infarct area was about 19.8% for the control group and about 19% for the group 2 and 4 wherein mix A and mix B+B respectively was used. Surprisingly, using the composition (B) according to the present invention, the infarct area was limited to 13.7% for the group 5 (mix B). Hence, the composition (B) according to the present invention was very efficient to treat infarct, such as myocardial infarction, compared to the other composition.

In addition, it was also surprisingly observed that when the injection of mix B followed the preliminary injection of mix A, the infarct area was further limited to the value of about 9.6%. This is a result which would not be expected based on the results observed for the other groups. Indeed, mix A used for the group 2 was almost inefficient alone. A synergistic effect was observed by using a pharmaceutical cocktail according to the present invention. This result was confirmed with histopathology and immunohistochemistry testing.

### Histopathology

Results were compiled separately for sections taken in the border zone or within the central areas of the infarct. Results for all the groups are listed in Table 3 below. Data showed in Table 3 are mean from heart slices analyzed for an animal of each group.

**Table 3**

| | Border zone | | Infarct center | |
|---|---|---|---|---|
| | Infarct/scar (%) | Transmurality (%) | Infarct/scar (%) | Transmurality (%) |
| Group 1 (Control) | 33.0 | 4.0 | 73.3 | 13.3 |
| Group 2 (Mix A) | 36.0 | 6.0 | 56.7 | 6.7 |
| Group 5 (Mix B) | 26.0 | 6.0 | 30.0 | 0.0 |
| Group 4 (Mix B+B) | 31.4 | 12.9 | 70.0 | 10.0 |
| Group 3 (Mix A+B) | 20.0 | 0.0 | 25.0 | 0.0 |

The ratio between the infarct and scar size represents the infarct size while the transmurality is a parameter establishing whether the infarct is strongly localized at the external surface of the myocardium or it extends throughout the internal surface of the myocardium. The higher is the transmurality value, the larger is the infarct.

With regard to the border zone of the infarct, Table 3 shows that mix A, mix B+B or control mix had almost no impact on the infarct size. In those cases, the ratio between the infarct and scar size ranged from 31.4% to 36.0%. On the contrary, when the composition according to the present invention is used (i.e. mix B), the ratio between infarct and scar size was surprisingly decreased to 26%. This value can be further decreased up to 20% when the pharmaceutical cocktail according to the present invention (i.e. mix A+B) was used. This experiment demonstrates that the present pharmaceutical composition and pharmaceutical cocktail are effective to treat heart disease. This was also confirmed when experiments were performed in the infarct zone.

Furthermore, it was surprisingly demonstrated that transmurality is reduced with mix B alone or mix A+B. This means that the composition (B) of the present invention alone or when combined with composition (A) allows limiting the expansion of the infarct to the external surface of the myocardium. This is another evidence that the pharmaceutical composition and the pharmaceutical cocktail according to the present invention are powerful compositions to treat heart diseases or troubles.

Hence, it is clear from the above-described experiments that both pharmaceutical composition and pharmaceutical cocktail according to the present invention are suitable for the treatment of heart failure secondary to myocardial ischemia, ischemia or myocardial infarction.

### Immunohistochemistry

Tests were performed to evaluate, within the infarct sections, the microvessel density (vWF-positive vessels/mm²), BrdU positive cells and c-kit positive cells. The quantification of microvessel density using von Willebrand factor (vWF) allows determining the amount of new blood vessels created in the infarct zone. BrdU positive cells tests represent the proliferation of cells, including cardiac cells. C-kit positive cells tests show the amount of CSCs within the selected infarct sections. Results are listed in Table 4. These testing were only performed for group 1 (Control group), group 3 (Mix A + B) and group 5 (Mix B).

**Table 4**

| | Group 1 (Control) | Group 3 (Mix A+B) | Group 5 (Mix B) |
|---|---|---|---|
| Microvessel Density (vWF-positive vessels/mm²) | 27.9 | 34.3 | 34.3 |
| BrdU positive cells (%) | 22.1 | 52.7 | 36.0 |
| c-kit positive cells (%) | 1.9 | 1.2 | 1.8 |

Results show that when compositions (A) and (B) in combination or composition (B) alone, according to the invention, are injected in the heart, they have great impact on cardiac stem cells stimulation or cardiac cells proliferation. Indeed, microvessel density is enhanced and new blood vessels were created upon stimulation with the present composition or present cocktail. Results obtained with groups 3 or 5 reached 34.2 and 34.3 respectively, compared to 27.9 for the control group. This is confirmed with BrdU positive cells test which shows that cells proliferation was enhanced with the composition of the present invention and that strong cellular activity was observed. When Mix B was injected, 36.0% of BrdU positive cells were observed compared to only 22.1 % for the control group. This clearly highlights that the pharmaceutical composition according to the present invention promotes cellular proliferation and thus the formation of new myocytes and vessels with the surrounding myocardium. This can be further enhanced when the pharmaceutical cocktail according to the present invention was used. A value of 52.7% was reached with such cocktail. Hence, both pharmaceutical composition and pharmaceutical cocktail according to the present invention are suitable for improving heart tissue regeneration.

The ability of the pharmaceutical cocktail to induce and to promote the CSCs activation and proliferation was confirmed with c-kit positive cells test. C-kit positive cells test allows demonstrating that resident CSCs are consumed since their amount has significantly decreased when mix A+B was used compared to the control group. Hence, the regenerated myocardial structures are originated from resident cardiac stem cells. The present composition and/or cocktail are effective for *in vivo* stimulation of resident cardiac stem cells.

## Claims

1. A human or veterinary pharmaceutical composition (B) suitable for the stimulation of stem cells, the pharmaceutical composition comprising: stem cells-stimulating-agents, and at least one pharmaceutically acceptable excipient, wherein the stem cells-stimulating-agents are TGFβ-1, BMP-4, FGF-2, IGF-1, Activin-A, Cardiotrophin 1, and Cardiogenol C; and wherein the pharmaceutical composition is free of alpha-thrombin.

2. The pharmaceutical composition of claim 1, wherein the composition further comprises a thrombin inhibitor.

3. A pharmaceutical composition according to claim 1 or claim 2, which further comprises at least one substance selected in the group consisting of growth factors, cytokines, hormones and combinations thereof, wherein the at least one substance is selected in the group consisting of
- Bone morphogenetic proteins (BMP) such as BMP-1, BMP-2, BMP-5, BMP-6;
- Epidermal growth factor (EGF);
- Erythropoietin (EPO);
- Fibroplast growth factors (FGF) such as FGF-1, FGF-4, FGF-5, FGF-12, FGF-13, FGF-15, FGF-20;
- Granulocyte-colony stimulating factor (G-CSF);
- Granulocyte-macrophage colony stimulating factor (GM-CSF);
- Growth differentiation factor-9 (GDF-9);
- Hepatocyte growth factor (HGF);
- Insuline-like growth factor (IGF) such as IGF-2;
- Myostatin (GDF-8);
- Neurotrophins such as NT-3, NT-4, NT-1 and Nerve growth factor (NGF);
- Platelet-derived growth factor (PDGF) such as PDGF-beta, PDGF-AA, PDGF-BB;
- Thrombopoietin (TPO);
- Transforming growth factor alpha (TGF-α)
- Transforming growth factors β, (TGF-β) such as TGF-β1, TGF-β2, TGF-β3;
- VEGF (Vascular endothelial growth factor) such as VEGF-A, VEGF-C;
- TNF-α, Leukemia inhibitory factor (LIF), interleukin 6 (IL-6), retinoic acid, SDF-1 (stromal cell-derived factor-1), BDNF (brain-derived neurotrophic factor), Periostin, Angiotensin II, Flt3 Ligand, Glial- Derived Neurotrophic Factor, Insulin-Like Growth Factor Binding Protein-3, Insulin- Like Growth Factor Binding Protein-5, Interleukin-3, Interleukin-8, Midkine, Progesterone, Putrescine, Stem Cell Factor, TGF-alpha, Wntl, Wnt3a, Wnt5a, caspase-4, chemokine ligand 1, chemokine ligand 2, chemokine ligand 5, chemokine ligand 7, chemokine ligand 11, chemokine ligand 20, haptoglobin, lectin, cholesterol 25-hydroxylase, syntaxin-8, syntaxin-11, ceruloplasmin, complement component 1, complement component 3, integrin alpha 6, lysosomal acid lipase 1, ϑ-2 microglobulin, ubiquitin, macrophage migration inhibitory factor, cofilin, cyclophillin A, FKBP12, NDPK, profilin 1, cystatin C, calcyclin, stanniocalcin-1, PGE-2, mpCCL2, IDO, iNOS, HLA-G5, M-CSF, angiopoietin, PIGF, MCP-1, extracellular matrix molecules, CCL2 (MCP-1), CCL3 (MIP-1α), CCL4 (MIP-1β), CCL5 (RANTES), CCL7 (MCP-3), CCL20 (MIP-3α), CCL26 (eotaxin-3), CX3CL1 (fractal kine), CXCL5 (ENA-78), CXCL11 (i-TAC), CXCL1 (GROα), CXCL2 (GROβ), CXCL8 (IL-8), CCL10 (IP- 10) and combinations thereof.

4. A pharmaceutical composition according to any of the previous claims, wherein stem cells to be stimulated are resident cardiac stem cells or circulating stem cells or injected stem cells.

5. A pharmaceutical composition according to any of the previous claims, which further comprises primary particles, wherein the primary particles encapsulate the stem cells-stimulating agents of said pharmaceutical composition (B).

6. A pharmaceutical composition according to claim 5 wherein the primary particles are selected from the group consisting of alginates, chitosan, dextran, cellulose, liposome, microspheres of polyesters, and nanospheres of polyesters. wherein said polyesters are selected from the group consisting of PLGA, polycaprolactone and copolyesters.

7. A pharmaceutical preparation comprising the pharmaceutical composition (B) according to any one of claims 1 to 3, and a composition (A) comprising at least one pharmaceutically active substance selected from the group consisting of insulin-like growth factor 1 (IGF-1), hepatocyte growth factor (HGF), and an HGF variant, wherein the HGF variant is NK1, 1K1, 1 K2, HP11, HP21, or a combination thereof.

8. A pharmaceutical preparation according to claim 7, wherein said composition A further comprises SCF-1 .

9. A pharmaceutical preparation according to any one of claims 7 or 8, wherein said composition (A) further comprises secondary particles, selected from the group consisting of alginates, chitosan, dextran, cellulose, liposome, or microspheres of polyesters, and or nanospheres of polyesters such as PLGA, polycaprolactone or copolyesters, wherein said secondary particles encapsulate said at least one pharmaceutically active substance.

10. A pharmaceutical preparation according to claim 9 wherein said secondary particles are configured to allow a delivery of the substance encapsulated therein before the delivery of the stem cells-stimulating agents encapsulated in primary particles.

11. The pharmaceutical composition (B) of claim 1 for use in the treatment of heart disease in a mammal, wherein the treatment comprises administering the pharmaceutical composition (B) to said mammal.

12. The pharmaceutical composition (B) for use in treatment of claim 11, wherein the treatment further comprises the preliminary administration of the composition (A) of claim 8.

13. The pharmaceutical composition (B) for use in treatment of claim 12, wherein at least one of the administrations is repeated.

14. The pharmaceutical composition (B) for use in treatment of claim 13, wherein the duration between two successive administrations of the pharmaceutical composition (B) is from 1 hour to 180 days.

15. The pharmaceutical composition (B) for use in treatment of claim 12, wherein the administration of pharmaceutical composition (B) is intracoronary, and the preliminary administration of composition (A) is intravenous.

## Patentansprüche

1. Human- oder tiermedizinische pharmazeutische Zusammensetzung (B), geeignet zum Stimulieren von Stammzellen, wobei die pharmazeutische Zusammensetzung Folgendes umfasst:
Stammzellen stimulierende Mittel und wenigstens einen pharmazeutisch unbedenklichen Hilfsstoff, wobei die Stammzellen stimulierenden Mittel TGFβ-1, BMP-4, FGF-2, IGF-1, Activin-A, Cardiotrophin 1 und Cardiogenol C sind und wobei die pharmazeutische Zusammensetzung frei von Alpha-Thrombin ist.

2. Pharmazeutische Zusammensetzung nach Anspruch 1, wobei die Zusammensetzung ferner einen Thrombinhemmer umfasst.

3. Pharmazeutische Zusammensetzung nach Anspruch 1 oder 2, die ferner wenigstens ein Mittel umfasst, ausgewählt aus der Gruppe bestehend aus Wachstumsfaktoren, Cytokinen, Hormonen und Kombinationen daraus, wobei das wenigstens eine Mittel ausgewählt ist aus der Gruppe bestehend aus den folgenden Mitteln:
- Knochenmorphogenetische Proteine (BMP), wie zum Beispiel BMP-1, BMP-2, BMP-5, BMP-6;
- Epidermaler Wachstumsfaktor (EGF);
- Erythropoietin (EPO);
- Fibroblasten-Wachstumsfaktoren (FGF), wie zum Beispiel FGF-1, FGF-4, FGF-5, FGF-12, FGF-13, FGF-15, FGF-20;
- Granulozyten-Kolonie stimulierender Faktor (G-CSF);
- Granulozyten-Makrophagen-Kolonie stimulierender Faktor (GM-CSF);
- Wachstums- und Differenzierungsfaktor-9 (GDF-9);
- Hepatozyten-Wachstumsfaktor (HGF);
- Insulinähnlicher Wachstumsfaktor (IGF), wie zum Beispiel IGF-2;
- Myostatin (GDF-8);
- Neurotrophine, wie zum Beispiel NT-3, NT-4, NT-1 und Nervenwachstumsfaktor (NGF);
- Thrombozytärer Wachstumsfaktor (PDGF), wie zum Beispiel PDGF-beta, PDGF-AA, PDGF BB;
- Thrombopoietin (TPO);
- Transformierender Wachstumsfaktor Alpha (TGF-α)
- Transformierende Wachstumsfaktoren β, (TGF-β), wie zum Beispiel TGF-β1, TGF-β2, TGF-β3;
- VEGF (vaskulärer endothelialer Wachstumsfaktor), wie zum Beispiel VEGF-A, VEGF-C;
- TNF-α, leukämiehemmender Faktor (LIF), Interleukin 6 (IL-6), Retinsäure, SDF-1 (Stromazellfaktor-1), BDNF (Neurotropher Hirnfaktor), Periostin, Angiotensin II, Flt3-Ligand, Neurotropher Glialzellenfaktor, insulinähnlichen Wachstumsfaktor bindendes Protein-3, insulinähnlichen Wachstumsfaktor bindendes Protein-5, Interleukin-3, Interleukin-8, Midkin, Progesteron, Putrescin, Stammzellfaktor, TGF-Alpha, Wnt1, Wnt3a, Wnt5a, Caspase-4, Chemokin-Ligand 1, Chemokin-Ligand 2, Chemokin-Ligand 5, Chemokin-Ligand 7, Chemokin-Ligand 11, Chemokin-Ligand 20, Haptoglobin, Lectin, Cholesterin-25-Hydroxylase, Syntaxin-8, Syntaxin-11, Ceruloplasmin, Komplementkomponente 1, Komplementkomponente 3, Integrin-Alpha-6, lysosomale saure Lipase 1, ϑ-2-Mikroglobulin, Ubiquitin, Makrophagenmigration inhibierender Faktor, Cofilin, Cyclophillin A, FKBP12, NDPK, Profilin 1, Cystatin C, Calcyclin, Stanniocalcin-1, PGE-2, mpCCL2, IDO, iNOS, HLA-G5, M-CSF, Angiopoietin, PIGF, MCP-1, extrazelluläre Matrixmoleküle, CCL2 (MCP-1), CCL3 (MIP-1α), CCL4 (MIP-1β), CCL5 (RANTES), CCL7 (MCP-3), CCL20 (MIP-3α), CCL26 (Eotaxin-3), CX3CL1 (Fraktalkin), CXCL5 (ENA-78), CXCL11 (i-TAC), CXCL1 (GROα), CXCL2 (GROß), CXCL8 (IL-8), CCL10 (IP-10) sowie Kombinationen aus diesen.

4. Pharmazeutische Zusammensetzung nach einem der vorhergehenden Ansprüche, wobei die zu stimulierenden Stammzellen residente Herzstammzellen oder zirkulierende Stammzellen oder injizierte Stammzellen sind.

5. Pharmazeutische Zusammensetzung nach einem der vorhergehenden Ansprüche, die ferner Primärpartikel umfasst, wobei die Primärpartikel die Stammzellen stimulierenden Mittel der pharmazeutischen Zusammensetzung (B) umschließen.

6. Pharmazeutische Zusammensetzung nach Anspruch 5, wobei die Primärpartikel ausgewählt sind aus der Gruppe bestehend aus Alginaten, Chitosan, Dextran, Cellulose, Liposom, Polyester-Mikrosphären und Polyester-Nanosphären, wobei die Polyester ausgewählt sind aus der Gruppe bestehend aus PLGA, Polycaprolacton und Copolyestern.

7. Pharmazeutisches Präparat, umfassend die pharmazeutische Zusammensetzung (B) nach einem der Ansprüche 1-3 sowie eine Zusammensetzung (A), welche wenigstens einen pharmazeutischen Wirkstoff umfasst, der ausgewählt ist aus der Gruppe bestehend aus insulinähnlichem Wachstumsfaktor 1 (IGF-1), Hepatozyten-Wachstumsfaktor (HGF) und einer HGF-Variante, wobei die HGF-Variante NK1, 1K1, 1 K2, HP11, HP21 oder eine Kombination aus diesen ist.

8. Pharmazeutisches Präparat nach Anspruch 7, wobei die Zusammensetzung A ferner SCF-1 umfasst.

9. Pharmazeutisches Präparat nach einem der Ansprüche 7 oder 8, wobei die Zusammensetzung (A) ferner Sekundärpartikel umfasst, ausgewählt aus der Gruppe bestehend aus Alginaten, Chitosan, Dextran, Cellulose, Liposom oder Polyester-Mikrosphären und/oder Polyester-Nanosphären, zum Beispiel aus PLGA, Polycaprolacton oder Copolyester, wobei die Sekundärpartikel wenigstens einen pharmazeutischen Wirkstoff umschließen.

10. Pharmazeutisches Präparat nach Anspruch 9, wobei die Sekundärpartikel konfiguriert sind, ein Verabreichen der davon umschlossenen Substanz zu ermöglichen, bevor die von den Primärpartikeln umschlossenen Stammzellen stimulierenden Mittel verabreicht werden.

11. Pharmazeutische Zusammensetzung (B) nach Anspruch 1 zum Einsatz bei der Behandlung von Herzkrankheit bei Säugetieren, wobei die Behandlung das Verabreichen der pharmazeutischen Zusammensetzung (B) an das Säugetier umfasst.

12. Pharmazeutische Zusammensetzung (B) zur Behandlung nach Anspruch 11, wobei die Behandlung ferner das vorausgehende Verabreichen der Zusammensetzung (A) nach Anspruch 8 umfasst.

13. Pharmazeutische Zusammensetzung (B) zum Einsatz in der Behandlung nach Anspruch 12, wobei wenigstens eine der Verabreichungen wiederholt wird.

14. Pharmazeutische Zusammensetzung (B) zum Einsatz in der Behandlung nach Anspruch 13, wobei der Zeitraum zwischen zwei aufeinanderfolgenden Verabreichungen der pharmazeutischen Zusammensetzung (B) zwischen 1 Stunde und 180 Tagen lang ist.

15. Pharmazeutische Zusammensetzung (B) zum Einsatz in der Behandlung nach Anspruch 12, wobei die Verabreichung der pharmazeutischen Zusammensetzung (B) intrakoronar ist und die vorausgehende Verabreichung der Zusammensetzung (A) intravenös ist.

## Revendications

1. Composition pharmaceutique à usage humain ou animal (B) appropriée pour la stimulation des cellules souches, la composition pharmaceutique comprenant : des agents de stimulation des cellules souches, et au moins un excipient pharmaceutiquement acceptable, les agents de stimulation des cellules souches étant TGFβ-1, BMP-4, FGF-2, IGF-1, l'activine-A, la cardiotrophine-1 et le cardiogénol-C ; et la composition pharmaceutique étant exempte d'alpha-thrombine.

2. Composition pharmaceutique selon la revendication 1, la composition comprenant en outre un inhibiteur de la thrombine.

3. Composition pharmaceutique selon la revendication 1 ou 2, comprenant en outre au moins une substance choisie dans le groupe constitué par les facteurs de croissance, les cytokines, les hormones et leurs mélanges, l'au moins une substance étant choisie dans le groupe constitué par :
- les protéines morphogénétiques osseuses (BMP) telles que BMP-1, BMP-2, BMP-5, BMP-6;
- le facteur de croissance de l'épiderme (EGF) ;
- l'érythropoïétine (EPO) ;
- les facteurs de croissance de fibroblastes (FGF) tels que FGF-1, FGF-4, FGF-5, FGF-12, FGF-13, FGF-15, FGF-20 ;
- le facteur de stimulation des colonies de granulocytes (G-CSF) ;
- le facteur de stimulation des colonies de macrophages-granulocytes (GM-CSF) ;
- le facteur-9 de croissance et de différenciation (GDF-9) ;
- le facteur de croissance des hépatocytes (HGF) ;
- le facteur de croissance apparenté à l'insuline (IGF) tel que IGF-2 ;
- la myostatine (GDF-8) ;
- les facteurs neurotrophiques tels que NT-3, NT-4, NT-1 et le facteur de croissance du tissu nerveux (NGF) ;
- le facteur de croissance dérivé des plaquettes (PDGF) tel que PDGF-bêta, PDGF-AA, PDGF-BB ;
- la thrombopoïétine (TPO) ;
- le facteur de croissance transformant alpha (TGF-α) ;
- les facteurs de croissance transformants β (TGF-β) tels que TGF-β1, TGF-β2, TGF-β3 ;
- VEGF (facteur de croissance endothélial vasculaire) tel que VEGF-A, VEGF-C ;
- TNF-α, le facteur d'inhibition de la leucémie (LIF), l'interleukine-6 (IL-6), l'acide rétinoïque, SDF-1 (facteur-1 dérivé de cellules stromales), BDNF (facteur neurotrophique dérivé du cerveau), la périostine, l'angiotensine II, le ligand Flt3, le facteur neurotrophique dérivé des cellules gliales, la protéine-3 de liaison des facteurs de croissance apparentés à l'insuline, la protéine-5 de liaison des facteurs de croissance apparentés à l'insuline, l'interleukine-3, l'interleukine-8, la midkine, la progestérone, la putrescine, le facteur des cellules souches, TGF-alpha, Wntl, Wnt3a, Wnt5a, la caspase-4, le ligand 1 de chémokine, le ligand 2 de chémokine, le ligand 5 de chémokine, le ligand 7 de chémokine, le ligand 11 de chémokine, le ligand 20 de chémokine, l'haptoglobine, la lectine, la cholestérol 25-hydroxylase, la syntaxine-8, la syntaxine-11, la céruloplasmine, la protéine 1 du complément, la protéine 3 du complément, l'intégrine alpha 6, la lipase acide lysosomale 1, la microglobuline θ-2, l'ubiquitine, le facteur d'inhibition de la migration des macrophages, le cofiline, la cyclophiline A, FKBP12, NDPK, la profiline 1, la cystatine C, la calcycline, la stanniocalcine-1, PGE-2, mpCCL2, IDO, iNOS, HLA-G5, M-CSF, l'angiopoïétine, PIGF, MCP-1, les molécules de matrice extracellulaire, CCL2 (MCP-1), CCL3 (MIP-1α), CCL4 (MIP-1β), CCL5 (RANTES), CCL7 (MCP-3), CCL20 (MIP-3α), CCL26 (éotaxine-3), CX3CL1 (kine fractale), CXCL5 (ENA-78), CXCL-11 (i-TAC), CXCL1 (GROα), CXCL2 (GROβ), CXCL8 (IL-8), CCL10 (IP-10) et leurs mélanges.

4. Composition pharmaceutique selon l'une quelconque des revendications précédentes, les cellules souches à stimuler étant des cellules souches cardiaques résidentes ou des cellules souches en circulation ou injectées.

5. Composition pharmaceutique selon l'une quelconque des revendications précédentes, comprenant en outre des particules primaires, les particules primaires encapsulant les agents de stimulation des cellules souches de ladite composition pharmaceutique (B).

6. Composition pharmaceutique selon la revendication 5, les particules primaires étant choisies dans le groupe constitué par les alginates, le chitosane, le dextrane, la cellulose, le liposome, les microsphères de polyesters et les nanosphères de polyesters, lesdits polyesters étant choisis dans le groupe constitué par le PLGA, la polycaprolactone et les copolyesters.

7. Préparation pharmaceutique comprenant la composition pharmaceutique (B) selon l'une quelconque des revendications 1 à 3, et une composition (A) comprenant au moins une substance pharmaceutiquement active choisie dans le groupe constitué par le facteur de croissance apparenté à l'insuline (IGF-1), le facteur de croissance des hépatocytes (HGF) et une variante du HGF, la variante du HGF étant NK1, 1 K1, 1 K2, HP11, HP21 ou un de leurs mélanges.

8. Préparation pharmaceutique selon la revendication 7, ladite composition A comprenant en outre SCF-1.

9. Préparation pharmaceutique selon l'une quelconque des revendications 7 ou 8, ladite composition (A) comprenant en outre des particules secondaires choisies dans le groupe constitué par les alginates, le chitosane, le dextrane, la cellulose, le liposome, ou les microsphères de polyesters et/ou les nanosphères de polyesters tels que le PLGA, la polycaprolactone ou les copolyesters, lesdites particules secondaires encapsulant ladite au moins une substance pharmaceutiquement active.

10. Préparation pharmaceutique selon la revendication 9, lesdites particules secondaires étant configurées de façon à permettre une libération de la substance encapsulée à l'intérieur avant la libération des agents de stimulation des cellules souches encapsulés dans les particules primaires.

11. Composition pharmaceutique (B) selon la revendication 1 pour une utilisation dans le traitement d'une maladie cardiaque chez un mammifère, le traitement comprenant l'administration de la composition pharmaceutique (B) audit mammifère.

12. Composition pharmaceutique (B) pour une utilisation dans le traitement selon la revendication 11, le traitement comprenant en outre l'administration préliminaire de la composition (A) selon la revendication 8.

13. Composition pharmaceutique (B) pour une utilisation dans le traitement selon la revendication 12, dans laquelle au moins une des administrations est répétée.

14. Composition pharmaceutique (B) pour une utilisation dans le traitement selon la revendication 13, la durée entre deux administrations successives de la composition pharmaceutique (B) étant comprise entre 1 heure et 180 jours.

15. Composition pharmaceutique (B) pour une utilisation dans le traitement selon la revendication 12, l'administration de la composition pharmaceutique (B) se faisant par voie intracoronaire, et l'administration préliminaire de la composition (A) se faisant par voie intraveineuse.
